# EUROPÄISCHE PATENTSCHRIFT

(11) **EP 0 905 119 B1**
(45) Veröffentlichungstag und Bekanntmachung des Hinweises auf die Patenterteilung: **13.02.2002**
(21) Anmeldenummer: 98117512.8
(22) Anmeldetag: 15.09.1998
(51) Int. Cl.: C07C 67/54, C07C 69/52, C07C 69/24

(54) **Verfahren zur Herstellung von hellfarbigen Fettsäureniedrigalkylestern mit vermindertem Gehalt an freiem und gebundenem Glycerin**
Process for preparing light-coloured lower alkyl esters of fatty acids with reduced content of free and combined glycerine
Procédé de fabrication d'esters faiblement colorés d'alcools alkyliques inférieurs et d'acides gras ayant une teneur réduite en glycérine libre ou combinée

(30) Priorität: 24.09.1997 DE 19742097
(43) Veröffentlichungstag der Anmeldung: 31.03.1999
(73) Patentinhaber: Cognis Deutschland GmbH, 40589 Düsseldorf (DE)
(72) Erfinder: Josten, Horst Dr., 40593 Düsseldorf (DE); Johannisbauer, Wilhelm Dr., 40699 Erkrath (DE); Kubersky, Hans Peter Dr., 42651 Solingen (DE); Schleper, Bernhard, 40764 Langenfeld (DE)

(56) Entgegenhaltungen:
- WO-A-95/27719
- DE-C- 4 209 779

## Beschreibung

Die Erfindung betrifft ein Verfahren zur Herstellung von Fettsäureniedrigalkylestern mit einem besonders niedrigen Gehalt an unerwünschten Verunreinigungen, bei dem man die Destillation in einer gepackten Vakuumkolonne durchführt, die Brüden niederschlägt und in eine Ester- und eine Glycerinphase trennt und die Esterphase wieder in die Kolonne zurückführt.

### Stand der Technik

Bei der Umesterung von pflanzlichen Ölen oder tierischen Fetten mit Methanol werden die im Öl oder Fett enthaltenen Triglyceride zu Methylestern und Glycerin umgesetzt. Nach Abtrennung von überschüssigem Methanol werden Methylester und Glycerin durch Schwerkraftabscheidung in einem Phasentrenngerät voneinander separiert (vgl. **Monographie "Fettalkohole, Rohstoffe, Verfahren und Verwendung", Henkel KGaA, 1981, S. 56/57**). Diese Trennung ist jedoch nicht vollständig, da bei diesem Prozessschritt noch Katalysator, z.B. Zinkacetat und Nebenbestandteile des Öles oder Fettes in der Mischung enthalten bleiben, die zu einer Zwischenphasenbildung und einer Emulgierung eines Teils des Glycerins im Methylester führen. Nach der Phasentrennung werden Methylester und Glycerin getrennt aufgearbeitet. Das Methylestergemisch wird je nach Art des Fettes oder Öles in einer Fraktionierkolonne in einen Vorschnitt und einen Hauptschnitt aufgetrennt. Anschließend wird der Hauptschnitt in einer Überkopfstufe destilliert, um Katalysator und schwersiedende Nebenprodukte als Rückstand abzutrennen. Bei der destillativen Aufarbeitung der Methylester gelangt das durch die unzureichende Phasentrennung noch vorhandene Glycerin auf Grund seines Siedeverhaltens in der Mischung zu geringen Teilen in den Vorschnitt, welcher im wesentlichen von kurzkettigen Methylestern gebildet wird. Der überwiegende Teil des Glycerins wird aber in der Überkopfstufe mit destilliert und findet sich so im Methylester-Endprodukt wieder.

Durch unvollständigen Umsatz der Triglyceride bei der Umesterung enthält der Roh-Methylester außerdem geringe Mengen von Mono- und Diglyceriden. Insbesondere die Monoglyceride gehen bei der Destillation über den Kopf der Kolonne in Abhängigkeit ihrer C-Kettenlänge mit in das Destillat über. Außerdem zeigt sich, dass unter den Temperaturbedingungen der Destillation infolge der Anwesenheit des Katalysators eine teilweise Umesterung der Monoglyceride zu Di- und Triglyceriden unter Freisetzung von Glycerin stattfindet. Sowohl das freie Glycerin als auch das in Form der Partialglyceride gebundene Glycerin führen zu einer Beeinträchtigung der Methylester-Qualität. Freies Glycerin führt zu einer Trübung der Methylester und erfordert eine Nachbehandlung, sofern die Ester als solche in den Handel gelangen. Sollen die Methylester indes zu den entsprechenden Fettalkoholen hydriert werden, führt ein Gehalt an freiem Glycerin zu einer signifikanten Verschlechterung der Katalysator-Standzeiten. Gebundenes Glycerin reagiert in der Hydrierung unter Bildung von Diolen ab, welche ihrerseits wieder eine Minderung der Fettalkohol-Qualität bewirken.

Neben freiem und gebundenem Glycerin führen auch Farbträger, die unter den Bedingungen der Überkopfdestillation gebildet werden, zu einer Verschlechterung der Methylester-Qualität. Dies gilt vor allem für Methylester, die nicht der Hydrierung zugeführt werden, sondern direkt als Endprodukte in den Handel gelangen. Hier können die erforderlichen Farbqualitäten nur erreicht werden, indem man im Rahmen der Destillation die Sumpftemperatur durch Absenken des Druckes am Kopf der Vakuumkolonne sowie durch Erhöhung der Rückstandsmengen möglichst niedrig hält. Dies ist freilich mit einer Reduzierung der Durchsatzmengen und damit bei verminderter Ausbeute mit einem Anstieg der Herstellkosten verbunden.

In der **DE-A1 3020612** ist die Möglichkeit des Auswaschens des Glycerins und der störenden Nebenbestandteile mit 50 bis 150 Gew.-% Wasser - bezogen auf den enthaltenen nicht umgesetzten niederen Alkohol - beschrieben. Gemäß der Lehre der **DE-A1 3107318** werden 30 bis 150 Gew.-% Wasser - bezogen auf den nicht umgesetzten niederen Alkoholnach der Umesterungsreaktion zugesetzt, um das Glycerin, den niederen Alkohol sowie störende Verunreinigungen, die die Farbe des Produktes beeinträchtigen, herauszuwaschen. Bei beiden Verfahren fallen jedoch größere Mengen methanolhaltiger Abwässer an. Eine andere Möglichkeit der Abtrennung des Glycerins ist in der **DE-A1 4209779** beschrieben. Dabei wird das Glycerin zunächst durch Schwerkraftabscheidung abgetrennt und anschließend noch in einem Separator einer Wäsche mit Wasser oder einer Pufferlösung unterworfen **[Fat.Sci.Technol. 96, 563 (1994)**]. Schließlich ist in diesem Zusammenhang aus der **DE-A1 19542197** ein mehrstufiges Umesterungsverfahren bekannt, bei dem man in der letzten Stufe der Umesterung der Reaktionsmischung 0,1 bis 3 Gew.-% Wasser zusetzt und die glycerinhaltige Phase vom Wertstoff abzieht. Alle diese Verfahren führen jedoch letztendlich nicht zu Produkten, die gleichzeitig hellfarbig und frei von Glycerin bzw. Partialglyceriden sind.

Aus der **WO 95/27719** (Procter & Gamble) ist ferner ein Verfahren zur Herstellung von Fettsäurepolyolestern bekannt, welche einen Gehalt an Monoglyceriden unterhalb vom 500 ppm sowie freies Glycerin unterhalb von 200 ppm aufweisen und dabei praktisch frei von Diund Triglyceriden sind. Hierbei wird zunächst ein Fettsäureglycerinester mit einem niederen primären Alkohol umgeestert, wobei im ersten Schritt ein Gemisch aus Fettsäureniedrigalkylestern, Partialglyceriden und Glycerin erhalten wird. Danach wird der Niedrigalkylester abgetrennt, aufgereinigt und schließlich mit einem geeigneten Polyol einer Umesterung unterworfen.

Die Aufgabe der vorliegenden Erfindung hat somit darin bestanden, ein Verfahren zur Herstellung von Fettsäureniedrigalkylestern, speziell Fettsäuremethylestern zur Verfügung zu stellen, bei dem besonders hellfarbige Produktqualitäten mit vermindertem Gehalt an freiem und gebundenem Glycerin erhalten werden.

### Beschreibung der Erfindung

Gegenstand der Erfindung ist ein Verfahren zur Herstellung von hellfarbigen Fettsäureniedrigalkylestern mit vermindertem Gehalt an freiem und gebundenem Glycerin der Formel **(I),**

**R**^{**1**}**CO-OR**^{**2**} **(I)**

in der R¹CO für einen linearen oder verzweigten, gesättigten oder ungesättigten Acylrest mit 6 bis 22 Kohlenstoffatomen und R² für einen linearen oder verzweigten Alkylrest mit 1 bis 4 Kohlenstoffatomen steht, bei dem man
(a) rohe Fettsäureniedrigalkylester in den Sumpfbereich einer Vakuumfraktionierkolonne einspeist, die zwei voneinander getrennte Segmente geordneter Stoffaustauschpackungen enthält, und dabei verdampft,
(b) die dabei erzeugten Brüden über den Kopf der Kolonne in mindestens einem nachgeschalteten Kondensator niederschlägt,
(c) das so gewonnene Kondensat in einem Phasentrenner in eine schwerere Glycerinphase und eine leichtere Niedrigalkylesterphase trennt,
(d) die leichtere Niedrigalkylesterphase abtrennt und als Rücklauf auf die obere Packung der Vakuumkolonne gibt, und
(e) den aufgereinigten Niedrigalkylester unterhalb der oberen Kolonnenpackung abzieht.

Der Kern der Erfindung besteht dabei darin, die aus dem Stand der Technik bekannte Überkopfdestillation durch eine Verfahrensstufe zu ersetzen, die aus einer Verschaltung einer Fraktionierkolonne mit verschiedenen Packungselementen zur Abreicherung von Glycerin, Monoglyceriden und Farb-trägern und eines Koaleszenzabscheiders zur Abtrennung des freien Glycerins besteht. Überraschenderweise wurde gefunden, dass mit den oben beschriebenen Verfahrensmaßnahmen Methylester erhalten werden, bei denen der Gehalt an freiem Glycerin bis unter die Löslichkeitsgrenze vermindert wird; eine Nachbehandlung der Produkte kann daher entfallen. Der Gehalt an Monoglyceriden und Farbträgern wird gleichfalls reduziert und im Rückstand angereichert. Dadurch werden bessere Farbqualitäten bei geringeren Rückstandsmengen und höheren Sumpftemperaturen als bei der bisherigen Verfahrensweise erzielt. Ein weiterer Vorteil des erfindungsgemäßen Verfahrens besteht darin, dass die Glycerinabtrennung und die Methylesterdestillation in einer Prozessstufe durchgeführt werden kann. Dadurch ist sichergestellt, dass auch bei der Destillation durch Umesterung der Monoglyceride freigesetztes Glycerin zuverlässig abgetrennt wird.

### Fettsäureniedrigalkylester

Typische Beispiele für geeignete Fettsäureniedrigalkylester, die im Sinne des erfindungsgemäßen Verfahrens als Rohstoffe in Betracht kommen, sind die Ethyl-, n-Propyl-, Isopropyl-, n-Butyl-, i-Butyl, sec-Butyl-, tert.-Butyl- und insbesondere Methylester von Fettsäuren mit 6 bis 22 und vorzugsweise 8 bis 18 Kohlenstoffatomen als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische Fettsäuremethylesterschnitte auf Basis von Kokosfettsäure oder Talgfettsäure eingesetzt. Die Fettsäureniedrigalkylester weisen dabei vorzugsweise einen Gehalt an freiem und gebundenem Glycerin von jeweils 1 bis 2 Gew.-% auf.

### Destillation und Phasentrennung

Die Destillation kann in an sich bekannter Weise durchgeführt werden, d.h. bei einer Temperatur im Bereich von 100 bis 250 °C und einem verminderten Druck von 100 Pa (1 mbar) bis 10.000 Pa (100 mbar). In einer bevorzugten Ausführungsform der Erfindung werden die die Fettsäureniedrigalkylester in den Sumpf der Vakuumkolonne eingespeist, welcher aus zwei getrennten Kammern gebildet wird, von denen zwei getrennte Fallfilmverdampfer über eine Zwangsumwälzung gespeist werden. Dies hat den Vorteil, dass die Temperaturbelastung der Alkylester bei der Destillation wesentlich vermindert und die Farbqualität weiter verbessert wird. Die Brüden aus den beiden Fallfilmverdampfern können dann ihrerseits unterhalb der unteren Stoffaustauschpackung in die Vakuumkolonne eingespeist werden. Das bei der Destillation am Kopf der Kolonne anfallende Destillat wird dann in mindestens einem, vorzugsweise zwei Kondensatoren niedergeschlagen und in den Phasentrenner geführt, in dem wiederum eine Trennung zwischen der schwereren Glycerin- und der leichteren Esterphase stattfindet. Während man das Glycerin als Wertprodukt ausschleust, wird ein Teilstrom des gereinigten Fettsäureniedrigalkalyesters, der noch 0,1 bis 0,2 Gew.-% Glycerin enthalten kann, als Rücklauf auf die obere Stoffaustauschpackung aufgegeben. Im Gegenstrom mit den aufsteigenden Brüden wird das Glycerin in der Flüssigkeit weiter abgereichert, so dass unterhalb der Kolonnenpackung der gereinigte Esterstrom abgezogen werden kann, bei dem der Glyceringehalt unterhalb der Löslichkeitsgrenze liegt. Da man in einer weiteren vorteilhaften Ausführungsform des Verfahrens gleichzeitig einen Teilstrom des destillierten Methylesters als Rücklauf auf die untere Packung geben kann, wird im Gegenstrom mit den aufsteigenden Brüden zusätzlich auch noch eine Abreicherung von schwersiedenden Monoglyceriden und Farbträgern erreicht. Die Schwersieder können dabei als Rückstand aus dem Verdampferkreislauf mindestens eines der beiden Fallfilmverdampfer ausgeschleust werden.

### Wärmerückgewinnung

Ein zusätzliches Anliegen der Erfindung besteht ferner darin, das Verfahren so kostengünstig wie möglich zu betreiben und hierzu gehört eine Wärmerückgewinnung auf möglichst hohem Niveau. Bei dem eingangs geschilderten erfindungsgemäßen Verfahren ist in diesem Zusammenhang zu beachten, dass eine Wärmerückgewinnung am Kopfkondensator nicht ohne Probleme möglich ist, da auf Grund der Fraktionierwirkung in der oberen Packung leichtsiedende Bestandteile der Mischung sich am Kopf der Kolonne anreichern, was schließlich zu einer Erniedrigung der Kopftemperatur führen kann. Eine Wärmerückgewinnung ist bei dieser Variante daher nur auf vergleichsweise niedrigem Niveau möglich.

In einer weiteren Ausgestaltungsform der Erfindung wird daher vorgeschlagen, mit Hilfe eines zwischen den beiden Stoffaustauschpackungen in der Vakuumkolonne angeordneten Teilkondensators einen Teil der Brüden niederzuschlagen und das Kondensat zusammen mit dem Kopfkondensat auf den Phasentrenner zu führen. Zwischen den beiden Packungen ist die Siedetemperatur in der Kolonne noch unverändert hoch, so dass eine Wärmerückgewinnung auf hohem Niveau möglich ist. In einer zweiten Variante wird ein Teil der Brüden als Teilstrom zwischen den beiden Stoffaustauschpackungen abgezogen, außerhalb der Vakuumkolonne in einem Totalkondensator niedergeschlagen und das Kondensat zusammen mit dem Kopfkondensat auf den Phasentrenner geführt. Dies hat gegenüber der zuvor geschilderten Variante den Vorteil, dass die Teilbrüden, die durch die obere Packung geführt werden, die gleiche niedrige Glycerinkonzentration aufweisen wie im Hauptverfahren.

### Beispiele

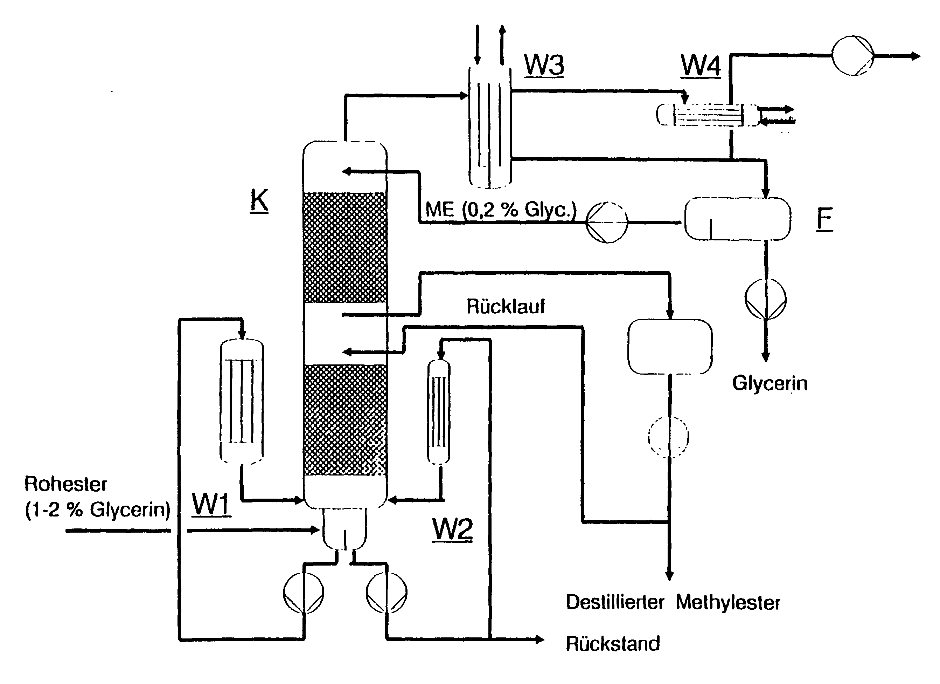

**Beispiel 1 (Abbildung 1).** Unraffinierter C_{12/18}-Kokosfettsäuremethylester mit einem Gehalt an freiem Glycerin von 1,5 Gew.-% und gebundenem Glycerin von 1,2 Gew.-% wurde nach Abtrennung niedrig-siedender Anteile in den Sumpfbereich einer unter Vakuum betriebenen Fraktionierkolonne **K** eingespeist. Der Sumpf bestand aus zwei getrennten Kammern, aus denen zwei getrennte Fallfilmverdampfer **W1** und **W2** über eine Zwangsumwälzung gespeist wurden. Die in den Verdampfern erzeugten Brüden wurden über die in die Kolonne eingebauten Segmente mit geordneter Stoffaustauschpackung geführt und im Hauptkondensator **W3** und dem nachgeschalteten Nachkondensator **W4** kondensiert. Das Kondensat wurde über den Phasentrenner **F** geführt, in dem das freie Glycerin als schwere Phase abgetrennt und ausgeschleust wurde. Die leichtere Methylesterphase wurde separat aus dem Phasentrenner abgezogen und als Rücklauf auf die obere Packung der Fraktionierkolonne gegeben, wobei der Glyceringehalt im Rücklauf noch 0,15 Gew.-% betrug. Im Gegenstrom mit den aufsteigenden Brüden wurde das Glycerin im gereinigten Methylester weiter abgereichert, der unterhalb der oberen Kolonnenpackung mit einem Glyceringehalt unterhalb der Löslichkeitsgrenze abgezogen wurde. Ein Teilstrom dieses destillierten Methylesters wurde als Rücklauf auf die untere Packung gegeben, wobei im Gegenstrom mit den aufsteigenden Brüden die Abreicherung von schwersiedenden Monoglyceriden und Farbträgern erreicht wurde. Gleichzeitig wurden die Schwersieder im Rückstand angereichert, der aus dem zweiten Verdampferkreislauf ausgeschleust wurde.

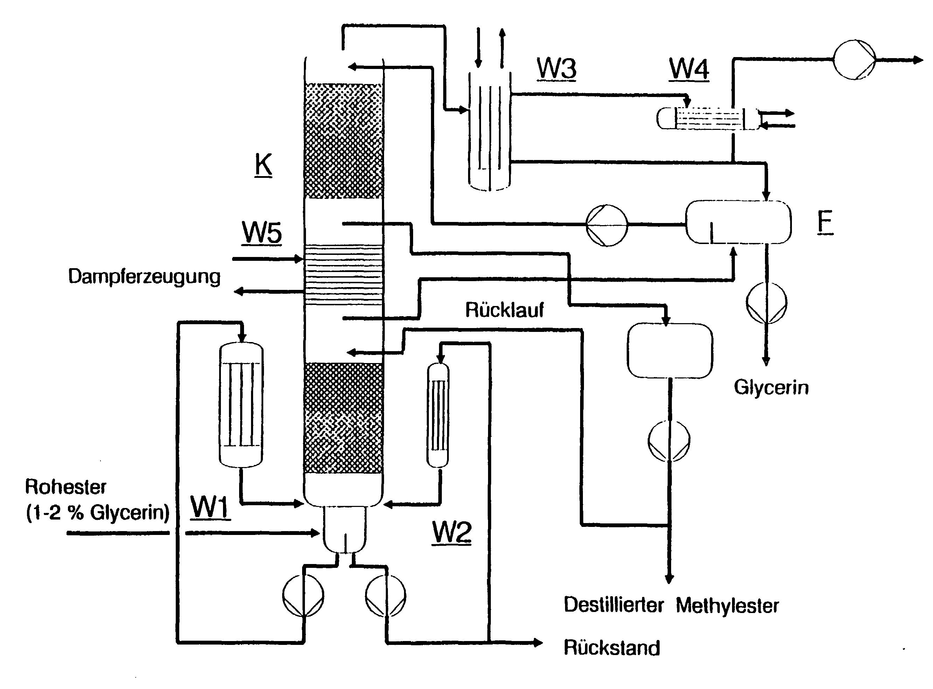

**Beispiel 2 (Abbildung 2).** Beispiel 2 unterscheidet sich von Beispiel 1 im wesentlichen dadurch, dass eine Wärmerückgewinnung auf hohem Niveau möglich ist. Hierzu wurde eine Kolonne **K** eingesetzt, welche zwischen den Kolonnenpackungen zusätzlich einen Teilkondensator **W5** enthielt. Ein Teil der Brüden wurde an dieser Stelle kondensiert und als Flüssigkeit aus der Kolonne abgezogen. Dieser Strom wurde zusammen mit dem Kondensat aus den Kondensatoren **W3** und **W4** über den Phasentrenner **F** geführt. Wiederum wurde das Glycerin als untere Phase abgeführt und ausgeschleust, während die Methylesterphase als Rücklauf auf die obere Kolonnenpackung zurückgegeben wurde.

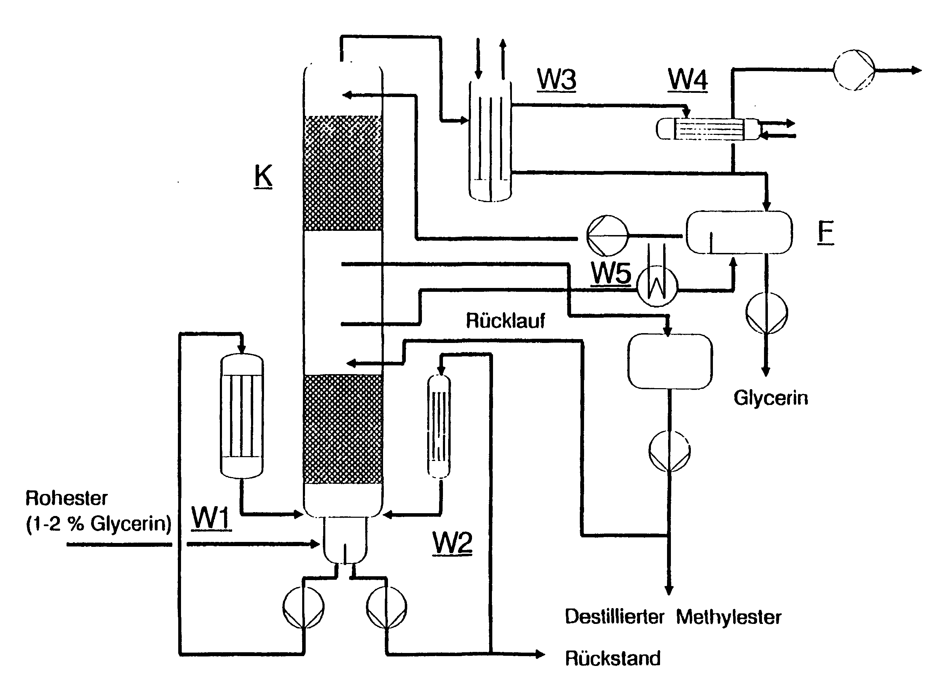

**Beispiel 3 (Abbildung 3).** Ähnlich wie in Beispiel 2, unterscheidet sich Beispiel 3 von Beispiel 1 im wesentlichen durch eine Wärmerückgewinnung auf hohem Niveau. Hierzu wurde ein dampfförmiger Seitenstrom an einer Stelle zwischen den beiden Packungen abgezogen und außerhalb der Kolonne in einem Totalkondensator **W5** kondensiert. Das Kondensat wurde wiederum zusammen mit dem Kopfkondensat aus den Kondensatoren **W3** und **W4** über den Phasentrenner **F** geführt. Auch hier wurde das Glycerin als schwerere Phase abgezogen und die Methylesterphase als Rücklauf auf den Kolonnenkopf zurückgegeben.

## Patentansprüche

1. Verfahren zur Herstellung von hellfarbigen Fettsäureniedrigalkylestern mit vermindertem Gehalt an freiem und gebundenem Glycerin der Formel **(I),**
**R**^{**1**}**CO-OR**^{**2**} **(I)**
in der R¹CO für einen linearen oder verzweigten, gesättigten oder ungesättigten Acylrest mit 6 bis 22 Kohlenstoffatomen und R² für einen linearen oder verzweigten Alkylrest mit 1 bis 4 Kohlenstoffatomen steht, bei dem man
(a) rohe Fettsäureniedrigalkylester in den Sumpfbereich einer Vakuumfraktionierkolonne einspeist, die zwei voneinander getrennte Segmente geordneter Stoffaustauschpackungen enthält, und dabei verdampft,
(b) die dabei erzeugten Brüden über den Kopf der Kolonne in mindestens einem nachgeschalteten Kondensator niederschlägt,
(c) das so gewonnene Kondensat in einem Phasentrenner in eine schwerere Glycerinphase und eine leichtere Niedrigalkylesterphase trennt,
(d) die leichtere Niedrigalkylesterphase abtrennt und als Rücklauf auf die obere Packung der Vakuumkolonne gibt, und
(e) den aufgereinigten Niedrigalkylester unterhalb der oberen Kolonnenpackung abzieht.

2. Verfahren nach Anspruch 1, **dadurch gekennzeichnet, dass** man technische Fettsäuremethylesterschnitte auf Basis von Kokosfettsäure oder Talgfettsäure einsetzt.

3. Verfahren nach den Ansprüchen 1 und 2, **dadurch gekennzeichnet, dass** man Fettsäureniedrigalkylester einsetzt, die einen Gehalt an freiem und gebundenem Glycerin von jeweils 1 bis 2 Gew.-% aufweisen.

4. Verfahren nach den Ansprüchen 1 bis 3, **dadurch gekennzeichnet, dass** man die Destillation bei einer Temperatur im Bereich von 100 bis 250 °C und einem verminderten Druck von 100 Pa (1 mbar) bis 10.000 Pa (100 mbar) durchführt.

5. Verfahren nach den Ansprüchen 1 bis 4, **dadurch gekennzeichnet, dass** man die Fettsäureniedrigalkylester in den Sumpf der Vakuumkolonne einspeist, welcher aus zwei getrennten Kammern gebildet wird, von denen zwei getrennte Fallfilmverdampfer über eine Zwangsumwälzung gespeist werden.

6. Verfahren nach Anspruch 5, **dadurch gekennzeichnet, dass** man die Brüden aus den beiden Fallfilmverdampfern unterhalb der unteren Stoffaustauschpackung in die Vakuumkolonne einspeist.

7. Verfahren nach den Ansprüchen 1 bis 6, **dadurch gekennzeichnet, dass** man einen Teilstrom des gereinigten Fettsäureniedrigalkalyesters als Rücklauf auf die untere Stoffaustauschpackung gibt.

8. Verfahren nach den Ansprüchen 1 bis 7, **dadurch gekennzeichnet, dass** man die Schwersieder als Rückstand aus dem Verdampferkreislauf mindestens eines der beiden Fallfilmverdampfer ausschleust.

9. Verfahren nach den Ansprüchen 1 bis 8, **dadurch gekennzeichnet, dass** man mit Hilfe eines zwischen den beiden Stoffaustauschpackungen in der Vakuumkolonne angeordneten Teilkondensators einen Teil der Brüden niederschlägt und das Kondensat zusammen mit dem Kopfkondensat auf den Phasentrenner führt.

10. Verfahren nach den Ansprüchen 1 bis 8, **dadurch gekennzeichnet, dass** man ein Teil der Brüden als Teilstrom zwischen den beiden Stoffaustauschpackungen abzieht, außerhalb der Vakuumkolonne in einem Totalkondensator niederschlägt und das Kondensat zusammen mit dem Kopfkondensat auf den Phasentrenner führt.

## Claims

1. A process for the production of light-coloured fatty acid lower alkyl esters with a reduced content of free and bound glycerol corresponding to formula (I):
R¹CO-OR² (I)
in which R¹CO is a linear or branched, saturated or unsaturated acyl group containing 6 to 22 carbon atoms and R² is a linear or branched alkyl group containing 1 to 4 carbon atoms,
**characterized in that**
(a) crude fatty acid lower alkyl esters are fed in at the bottom of a vacuum fractionating column comprising two separate segments of stacked material transfer packings and are evaporated,
(b) the vapours produced are condensed via the head of the column in at least one following condenser,
(c) the condensate obtained is separated in a phase separator into a heavier glycerol phase and a lighter lower alkyl ester phase,
(d) the lighter lower alkyl ester phase is removed and fed as reflux to the upper packing of the vacuum column
(e) the purified lower alkyl ester is removed below the upper column packing.

2. A process as claimed in claim 1, **characterized in that** technical fatty acid methyl ester cuts based on coconut fatty acid or tallow fatty acid are used.

3. A process as claimed in claims 1 and 2, **characterized in that** fatty acid lower alkyl esters containing 1 to 2% by weight of free glycerol and 1 to 2% by weight of bound glycerol.

4. A process as claimed in claims 1 to 3, **characterized in that** the distillation is carried out at a temperature of 100 to 250°C under a reduced pressure of 100 Pa (1 mbar) to 10,000 Pa (100 mbar).

5. A process as claimed in claims 1 to 4, **characterized in that** the fatty acid lower alkyl esters are fed in at the bottom of the vacuum column which is formed by two separate compartments from which two separate falling film evaporators are fed via a forced circulation system.

6. A process as claimed in claim 5, **characterized in that** the vapours from the two falling film evaporators are fed into the vacuum column below the lower material transfer packing.

7. A process as claimed in claims 1 to 6, **characterized in that** part of the purified fatty acid lower alkyl ester is delivered as reflux to the lower material transfer packing.

8. A process as claimed in claims 1 to 7, **characterized in that** the high boilers are removed as residue from the evaporator circuit of at least one of the two falling film evaporators.

9. A process as claimed in claims 1 to 8, **characterized in that** the vapours are partly condensed by means of a partial condenser arranged between the two material transfer packings in the vacuum column and the condensate is passed with the head condensate to the phase separator.

10. A process as claimed in claims 1 to 8, **characterized in that** the vapours are partly removed between the two material transfer packings, condensed in a total condenser outside the vacuum column and the condensate is passed with the head condensate to the phase separator.

## Revendications

1. Procédé pour la production d'esters faiblement colorés d'alcools alkyliques inférieurs et d'acides gras, à teneur réduite en glycérol libre et en glycérol lié, de formule (I)
R¹CO-OR² (I)
dans laquelle R¹CO représente un radical acyle linéaire ou ramifié, saturé ou insaturé, ayant de 6 à 22 atomes de carbone, et R² représente un radical alkyle linéaire ou ramifié ayant de 1 à 4 atomes de carbone, dans lequel
(a) on introduit des esters bruts d'alcools alkyliques inférieurs et d'acides gras dans la zone du pied d'une colonne de fractionnement sous vide qui comporte deux segments, séparés l'un de l'autre, de garnissages disposés pour l'échange de substances, et on les vaporise dans cette zone,
(b) les vapeurs chaudes ainsi produites par la tête de la colonne sont condensées dans au moins un condenseur disposé en aval,
(c) le condensat ainsi obtenu est fractionné, dans un séparateur de phases, en une phase plus lourde de glycérol et une phase plus légère d'ester alkylique inférieur,
(d) on sépare la phase plus légère d'ester alkylique inférieur et on l'envoie, en tant que courant de recyclage, sur le garnissage supérieur de la colonne à vide, et
(e) on soutire l'ester alkylique inférieur purifié au-dessous du garnissage supérieur de la colonne.

2. Procédé selon la revendication 1,
**caractérisé en ce qu'**
on utilise des fractions industrielles d'esters méthyliques d'acides gras à base d'acide gras de coco ou d'acide gras de suif.

3. Procédé selon les revendications 1 et 2,
**caractérisé en ce qu'**
on utilise des esters alkyliques inférieurs d'acides gras qui ont une teneur en glycérol libre et glycérol lié, respectivement, de 1 à 2 % en poids.

4. Procédé selon les revendications 1 à 3,
**caractérisé en ce qu'**
on effectue la distillation à une température dans la plage de 100 à 250°C et sous une pression réduite dans la plage allant de 100 Pa (1 mbar) à 10 000 Pa (100 mbars).

5. Procédé selon les revendications 1 à 4,
**caractérisé en ce qu'**
on introduit les esters alkyliques inférieurs d'acides gras au pied de la colonne à vide, qui est constitué de deux chambres séparées, par lesquelles sont alimentés par une recirculation forcée deux évaporateurs séparés à film descendant.

6. Procédé selon la revendication 5,
**caractérisé en ce que**
les vapeurs chaudes provenant des deux évaporateurs à film descendant sont introduites au-dessous du garnissage inférieur pour échange de substances dans la colonne à vide.

7. Procédé selon les revendications 1 à 6,
caractérisè en ce qu'
on envoie sur le garnissage inférieur pour échange de substances un courant partiel de l'ester purifié d'alcool inférieur et d'acide gras, en tant que courant de recyclage.

8. Procédé selon les revendications 1 à 7,
**caractérisé en ce qu'**
on envoie les composants à haut point d'ébullition, en tant que résidu du circuit d'évaporateur, à au moins l'un des deux évaporateurs à film descendant.

9. Procédé selon les revendications 1 à 8,
**caractérisé en ce qu'**
une partie des vapeurs chaudes est condensée à l'aide d'un condenseur partiel disposé entre les deux garnissages pour échange de substances dans la colonne à vide, et le condensat est envoyé, conjointement avec le condensat de tête, au séparateur de phases.

10. Procédé selon les revendications 1 à 8,
**caractérisé en ce qu'**
une partie des vapeurs chaudes est soutirée en tant que courant partiel entre les deux garnissages pour échange de substances, condensée en dehors de la colonne à vide, dans un condenseur total, et le condensat est envoyé, conjointement avec le condensat de tête, au séparateur de phases.
